# EUROPÄISCHE PATENTANMELDUNG

(11) **EP 1 498 762 A1**
(43) Veröffentlichungstag der Anmeldung: **19.01.2005**
(21) Anmeldenummer: 04016741.3
(22) Anmeldetag: 15.07.2004
(51) Int. Cl.: G02B 21/22

(54) **Mikroskop**

(30) Priorität: 17.07.2003 DE 10332602; 17.07.2003 DE 10332603
(71) Anmelder: Leica Microsystems (Schweiz) AG, 9435 Heerbrugg (CH)
(72) Erfinder: Sander, Ulrich, 9445 Rebstein (CH)
(74) Vertreter: Hössle Kudlek & Partner

(57) **Zusammenfassung**

Mikroskop, insbesondere Stereomikroskop, mit einem Hauptobjektiv (2), einem diesem nachgeschalteten Vergrößerungssystem (7) und einer Zusatzoptik (30, 32a) zur Durchführung intraokularer Chirurgie, wobei die Zusatzoptik wenigstens eine dem Hauptobjektiv (2) vorgeschaltete Ophthalmoskopierlinse (30) und wenigstens eine dem Hauptobjektiv (2) nachgeschaltete optische Komponente (32a) zur Bereitstellung einer Brechung und Fokussierung eines das Hauptobjektiv (2) durchsetzenden Beobachtungsstrahlenganges aufweist, und die optische Achse (12d) des dem Hauptobjektiv nachgeschalteten Vergrößerungssystems (7) im wesentlichen senkrecht zu der optischen Achse (11) des Hauptobjektivs (2) verläuft.

## Beschreibung

Die vorliegende Erfindung betrifft ein Mikroskop, insbesondere ein Stereomikroskop, nach dem Oberbegriff des Patentanspruchs 1.

Ophthalmologische Mikroskope sind an sich bekannt. Sie weisen ein Hauptobjektiv, ein diesem nachgeschaltetes Vergrößerungssystem und ein Binokularsystem mit Okularen auf. Zur Bereitstellung eines Stereomikroskops kann in einem beispielsweise als Zoom-System ausgestalteten Vergrößerungssystem eine Aufspaltung eines das Hauptobjektiv durchsetzenden Strahlengangs in eine Anzahl von Strahlengängen durchgeführt werden. Ferner sind ophthalmologische Mikroskope bekannt, welche eine simultane Betrachtung eines Objektes durch einen ersten Benutzer (Hauptoperateur) und einen zweiten Benutzer (Assistent) gestatten.

Für die intraokulare Chirurgie, beispielsweise um den Fundus oder fundusnahe Glaskörperbereiche eines menschlichen Auges mikroskopisch betrachten zu können, werden Zusatzoptiken an den Stereomikroskopen benötigt. Diese bestehen aus Linsen, die dem Hauptobjektiv (objektseitig) vorgeschaltet sind. Da sie somit außerhalb des Mikroskopkörpers positioniert sind, erweisen sie sich für den operierenden Chirurgen als sehr störend, da sie den freien Arbeitsabstand einschränken. Ferner sind derartige Zusatzoptiken sehr schmutzempfindlich, so dass auch die Sterilität derart angeordneter Zusatzoptiken zu wünschen übrig lässt.

In dem Prospekt SDI II, BIOM II der Oculus Optikgeräte GmbH aus dem Jahre 1998 sowie der US 4,856,872 ist eine derartige Zusatzoptik beschrieben. Man erkennt, dass dort ein als Biom-System (Biom: engl. Binokular Indirect Ophthalmomicroscope) ausgebildetes Linsensystem nach unten in das Operationsfeld hineinragt und aus diesem Grunde durch die Tätigkeit des Operateurs leicht verschmutzt werden kann. Hierbei sind sowohl die nah am Objekt angeordnete Linse (Ophthalmoskopierlinse) als auch die näher am Hauptobjektiv angeordnete Linse (Reduktionslinse) verschmutzbar. Im Falle länger andauernder Operationen müssen daher beide Linsen dieser vorgeschalteten Zusatzoptik in regelmäßigen Abständen gesäubert werden, was mit relativ großem Aufwand verbunden ist.

Ein mit einer derartigen Zusatzoptik ausgebildetes Mikroskop ist beispielsweise in dem deutschen Gebrauchsmuster G 9415219.5 beschrieben.

Aus der DE 41 14 646 C2 ist eine Lösung bekannt, bei der ein Ophthalmologie-Vorsatz für ein Operationsmikroskop in einem Vorsatzgehäuse untergebracht ist, welches bezüglich des Hauptobjektivs seitlich positionierbar ist. Der Vorsatz weist eine Ophthalmoskopierlinse, ein optisches System zur Bildaufrichtung und eine verschiebbare Linse zur Fokussierung auf. Die Anordnung dieser beiden Linsen bzw. Linsensysteme in einem Gehäuse wird als relativ aufwendig angesehen. Ferner erweist sich in der Praxis ein derartiges Gehäuse für den Operateur als störend.

Aus der DE 3528356 A1 ist eine Vorrichtung für die Untersuchung und Chirurgie der vorderen und hinteren Augenabschnitte bekannt. Bei dem dort beschriebenen Gerät für die Untersuchung und Chirurgie des Auges ist ein ophthalmologisches Objektiv mit einem Operationsmikroskop kombiniert, dessen Hauptobjektiv mit einem optischen System variabler Schnitt- und Brennweite kombiniert ist.

Ein Ziel der Erfindung ist die Bereitstellung eines Mikroskops mit einer insbesondere intraokulare Chirurgie ermöglichenden, dem Hauptobjektiv vorgeschalteten Zusatzoptik, bei dem ein Verschmutzen dieser Zusatzoptik während der Durchführung von Operationen weitgehend vermieden werden kann, wobei die Zusatzoptik möglichst klein bauen und somit den Operateur möglichst wenig behindern soll. Die Strahlengänge innerhalb des Mikroskops sollen hierbei möglichst einfach sein.

Das erfindungsgemäße Mikroskop weist wenigstens eine dem Hauptobjektiv vorgeschaltete Linse, und wenigstens eine dem Hauptobjektiv nachgeschaltete Komponente, insbesondere wenigstens eine Linse auf. Durch diese Anordnung eines Teils der Zusatzoptik hinter dem Hauptobjektiv, d. h. bezüglich des Hauptobjektivs beobachterseitig im Mikroskopgehäuse, kann eine Verschmutzung dieses Teils der Zusatzoptik während einer Operation wirksam vermieden werden. Erfindungsgemäß verbleibt objektseitig des Hauptobjektivs lediglich die relativ kleine Ophthalmoskopierlinse, welche bereits aufgrund ihrer (geringen) Größe sehr leicht zu reinigen ist. Hierdurch ist der Reinigungsaufwand, beispielsweise während einer Operation, gegenüber herkömmlichen Lösungen vermindert. Das erfindungsgemäße Mikroskop zeichnet sich ferner dadurch aus, dass die optische Achse des dem Hauptobjektiv nachgeschalteten Vergrößerungssystems im wesentlichen senkrecht zu der optischen Achse des Hauptobjektivs verläuft. Mit dieser Maßnahme kann die vertikale Erstreckung des Mikroskops, und damit die ergonomische Bauhöhe in wirksamer Weise minimiert werden.

Vorteilhafte Ausgestaltungen der Erfindung sind Gegenstand der Unteransprüche.

Es ist bevorzugt, dass die dem Hauptobjektiv vorgeschaltete Ophthalmoskopierlinse und die dem Hauptobjektiv nachgeschaltete optische Komponente entlang der optischen Achse des Hauptobjektivs angeordnet sind. Mit dieser Maßnahme können Umlenkungen eines konvergenten Strahlenganges zwischen der Ophthalmoskopierlinse und dem Hauptobjektiv vermieden werden, da nach dem Durchgang durch das Hauptobjektiv und der dem Hauptobjektiv nachgeschalteten Komponente die vom Objekt ausgehenden Strahlengänge im wesentlichen parallel verlaufen. Zur Umlenkung derartiger parallel verlaufender Strahlengänge können relativ klein ausgebildete Umlenkelemente verwendet werden, ohne dass es zu Vignettierungen kommt. Die Umlenkung von konvergenten Strahlengängen erfordert hingegen, sollen Vignettierungen vermieden werden, relativ große Umlenkelemente.

Es ist ferner bevorzugt, dass das erfindungsgemäß vorgesehene Vergrößerungssystem wenigstens zwei, insbesondere drei oder vier Beobachtungskanäle aufweist. Insbesondere die Bereitstellung eines mit vier Beobachtungskanälen ausgebildeten Vergrößerungssystems hinter einem Inverter-System (beobachterseitig) ermöglicht eine sehr unaufwendige stereoskopische Beobachtung durch zwei Beobachter, d.h. einen Hauptoperateur und einen Assistenten. Durch eine derartige Anordnung des Vergrößerungssystems hinter einem Inverter-System ist es lediglich notwendig, für den Hauptoperateur und den Assistenten ein einziges Inverter-System vorzusehen. Es ist ebenfalls denkbar, ein mit einer Mehrzahl von Vergrößerungs-Kanälen ausgebildetes Vergrößerungssystem vor dem Inverter-System (d.h. objektseitig) zu positionieren.

Zweckmäßigerweise ist die erfindungsgemäß vorgesehene Zusatzoptik zur Durchführung intraokularer Chirurgie als BIOM-System ausgebildet. Derartige Systeme wenden die Prinzipien der indirekten Ophthalmoskopie an und ermöglichen eine sehr weitwinklige Beobachtung bspw. des Augenfundus. Bei Verwendung derartiger Systeme kann das Auge frei gedreht werden, so daß auch die Peripherie des Fundus gut beobachtet werden kann.

Gemäß einer bevorzugten Ausführungsform des erfindungsgemäßen Mikroskops ist die dem Hauptobjektiv vorgeschaltete Linse der Zusatzoptik, insbesondere durch Verschwenkung, und/oder die dem Hauptobjektiv nachgeschaltete optische Komponente, insbesondere durch Verschiebung, aus dem Beobachtungsstrahlengang des Hauptobjektivs entfernbar. Da somit insbesondere die gesamte Zusatzoptik aus dem Beobachtungsstrahlengang entfernbar ist, ist ein derart ausgestattetes Mikroskop auch für normale ophthalmologische Anwendungen, bei der eine derartige Zusatzoptik nicht benötigt wird, einsetzbar.

Vorteilhafterweise ist die dem Hauptobjektiv nachgeschaltete optische Komponente der Zusatzoptik in Richtung des sie beaufschlagenden Beobachtungsstrahlenganges hin- und herverschiebbar. Mit dieser Maßnahme ist eine Fokussierung des Beobachtungsstrahlenganges, welche aufgrund des Vorhandenseins der Zusatzoptik notwendig sein kann, in einfacher Weise möglich. Dies bedeutet beispielsweise, dass es mit dieser Linse möglich ist, auf den interessierenden Abschnitt eines Auges zu fokussieren, ohne am eigentlichen Operationsmikroskop etwas verändern zu müssen.

Vorteilhafterweise ist ein Umlenkelement vorgesehen, das einen entlang der optischen Achse des Hauptobjektivs verlaufenden Strahlengang in eine erste, sich im wesentlichen senkrecht zur optischen Achse des Hauptobjektivs erstreckende Mikroskopebene umlenkt.

Gemäß einer bevorzugten Ausführungsform sind zwei Umlenkelemente vorgesehen, mittels der ein in der ersten Mikroskopebene verlaufender Strahlengang in eine zweite Mikroskopebene ablenkbar ist, wobei wenigstens eines der Umlenkelemente mit einer Brechkraft ausgebildet ist. Derartige Umlenkelemente können in einfacher und kostengünstiger Weise ein Inverter-System zur Verfügung stellen. Die erste und die zweite Mikroskopebene verlaufen zweckmäßigerweise horizontal.

Gemäß einer weiteren bevorzugten Ausführungsform des erfindungsgemäßen Mikroskops ist ein Umlenkelement zwischen dem Hauptobjektiv und der dem Hauptobjektiv nachgeschalteten optischen Komponente der Zusatzoptik vorgesehen, so dass der Strahlengang durch die dem Hauptobjektiv nachgeschaltete Komponente der Zusatzoptik unter einem Winkel, insbesondere senkrecht, zu dem Strahlengang durch die dem Hauptobjektiv vorgeschaltete Linse der Zusatzoptik verläuft. Mit dieser Maßnahme ist es beispielsweise möglich, die dem Hauptobjektiv nachgeschaltete Komponente der Zusatzoptik in einen im Wesentlichen waagerecht bzw. horizontal verlaufenden Strahlengang einzubringen, wodurch die Bauhöhe des Mikroskops sehr klein gehalten werden kann.

Es ist ferner gemäß einer weiteren bevorzugten Ausführungsform des erfindungsgemäßen Mikroskops vorgesehen, das zwischen dem Hauptobjektiv und der dem Hauptobjektiv nachgeschalteten optischen Komponente der Zusatzoptik vorgesehene Umlenkelement mit einer Brechkraft auszubilden. Mit dieser Maßnahme ist es möglich, die durch die nachgeschaltete Komponente bereitzustellende Brechkraft wenigstens teilweise auf das Umlenkelement zu verlagern. Hierdurch erhält man bei der konstruktiven Ausbildung der nachgeschalteten optischen Komponente größere Freiheiten. Das Umlenkelement ist hier zweckmäßigerweise als Hohlspiegel oder als entsprechend gekrümmte Flächen aufweisendes Prisma ausgebildet. Auch die Umlenkelemente zur Umlenkung des Strahlenganges von der ersten in die zweite Mikroskopebene können, wie erwähnt, mit einer Brechkraft ausgebildet sein.

Gemäß einer weiteren bevorzugten Ausführungsform des erfindungsgemäßen Mikroskops ist eine elektromechanische Verbindung der Ophthalmoskopierlinse und der dem Hauptobjektiv nachgeschalteten optischen Komponente der Zusatzoptik zum gemeinsamen Ausschwenken bzw. Verschieben dieser Komponenten vorgesehen. In diesem Zusammenhang ist es beispielsweise möglich, jede der Komponenten mit einem Elektromotor auszubilden, wobei die beiden Elektromotoren gemeinsam bzw. in Abhängigkeit von einander steuerbar sind.

Zweckmäßigerweise sind die Brechkraft und der Verschiebebereich der dem Hauptobjektiv nachgeschalteten optischen Komponente so gewählt, dass ein Fokussierausgleich vom Ort der Beobachtung des Objektes bei Nichtverwendung der Ophthalmoskopierlinse bis hin zum Ort der Zwischenabbildung, die sich bei Verwendung der Ophthalmoskopierlinse ergibt, möglich ist. Mit dieser Maßnahme kann gewährleistet werden, dass keine Verschiebung des Mikroskopkörpers während einer Fokussierung auf ein Objekt notwendig ist, da diese Fokussierung lediglich durch Verschiebung der dem Hauptobjektiv nachgeschalteten optischen Komponente bewerkstelligt werden kann.

Es ist ferner bevorzugt, das erfindungsgemäße Mikroskop mit einem Autofokussystem auszubilden. Mit einem derartigen Autofokussystem lässt sich die Verschiebung der dem Hauptobjektiv nachgeschalteten optischen Komponente parallel zur optischen Achse des Hauptobjektivs in einfacher Weise steuern bzw. regeln. Derartige Autofokussysteme sind an sich bekannt. Beispielsweise erfolgt eine Autofokussierung über die Einkopplung eines Laserstrahls nach dem Triangulationsprinzip.

Die Erfindung wird nun anhand der beigefügten Zeichnung weiter erläutert. In dieser zeigt:
- Figur 1: ein mit einer herkömmlichen Zusatzoptik zur Durchführung intraokularer Chirurgie ausgestattetes Mikroskop in schematischer seitlicher Schnittansicht und,
- Figur 2: eine bevorzugte Ausführungsform des erfindungsgemäßen Mikroskops in einer der Figur 1 entsprechenden Ansicht.

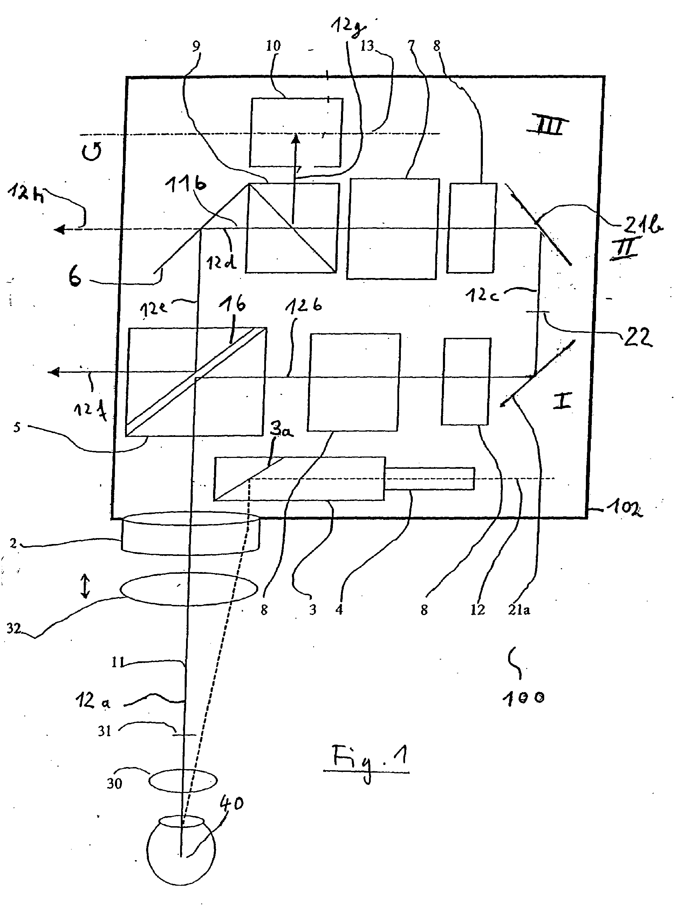

Ein Stereomikroskop mit einer herkömmlichen Zusatzoptik zur Durchführung intraokularer Chirurgie ist in Figur 1 insgesamt mit 100 bezeichnet. Das Stereomikroskop weist einen Mikroskopkörper 102 auf, in dem als wesentliche optische Komponenten ein Hauptobjektiv 2 und ein insbesondere als Zoom-System ausgebildetes Vergrößerungssystem 7 vorgesehen sind.

Das Mikroskop 100 weist ferner als Planspiegel ausgebildete Umlenkelemente 5, 21a, 21b auf. Mittels dieser Umlenkelemente sind von einem zu beobachtenden Objekt 40 ausgehende Beobachtungsstrahlen 12a - 12h, welche zunächst im wesentlichen (bei 12a) in vertikaler Richtung das Hauptobjektiv 2 entlang dessen optischer Achse 11 durchlaufen, in zwei im wesentlichen horizontal verlaufende Mikroskopebenen I, II umlenkbar (bei 12b, 12d). Man erkennt, daß das Vergrößerungssystem 7 in der dargestellten Ausführungsform in der zweiten Ebene II angeordnet ist.

Objektseitig bezüglich des Vergrößerungssystems 7 sind, wahlweise in der ersten und/oder zweiten Ebene I, II, optische Zusatzkomponenten, hier insgesamt mit 8 bezeichnet, wie beispielsweise Filter, Lasershutter , optische Teiler oder SDI-Einrichtungen (Stereoskopische Diagonal-Inverter) vorgesehen.

Das dargestellte Mikroskop 100 ist zur simultanen Beobachtung des Objekts 40 durch einen Hauptoperateur und einen Assistenten ausgelegt. Zu diesem Zweck ist in der zweiten Mikroskopebene II ein Umlenkelement bzw. eine Auskopplungseinrichtung 9 vorgesehen, welche die Auskopplung des Beobachtungsstrahlenganges 12g für den Assistenten bezüglich des Beobachtungsstrahlenganges 12d für den Hauptoperateur bewirkt. Die Beobachtung des Objektes 40 durch den Assistenten erfolgt in einer dritten Mikroskopebene III, wie weiter unten näher beschrieben wird.

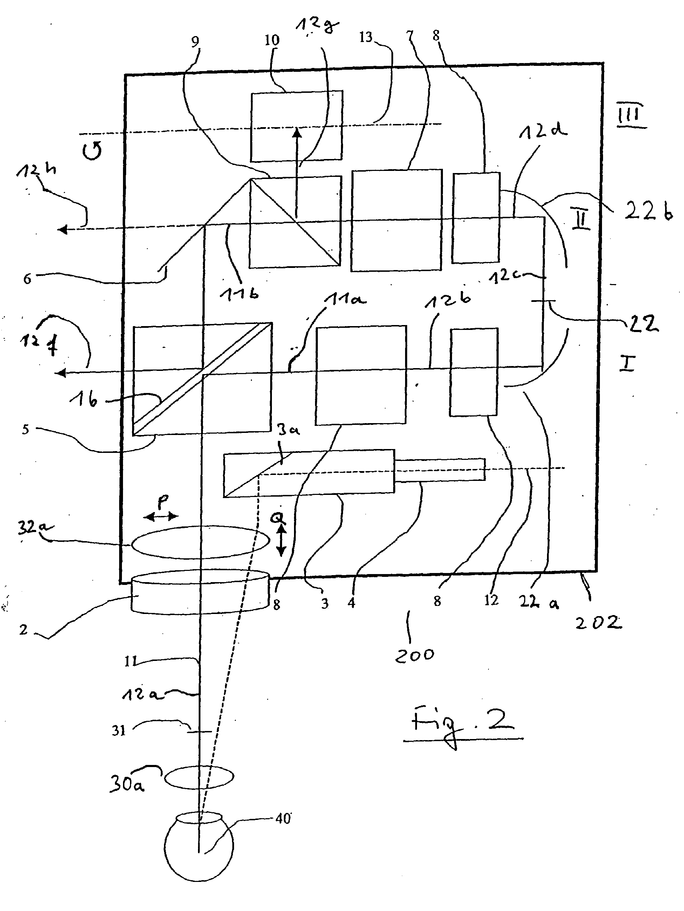

Die stereoskopische Aufspaltung des das Hauptobjektiv 2 beaufschlagenden einheitlichen Strahlengangs 12a kann in an sich bekannter Weise an beliebiger Stelle innerhalb des Mikroskopgehäuses 102 erfolgen. Zweckmäßigerweise erfolgt die stereoskopische Aufspaltung mittels des Vergrößerungssystems 7, welches beispielsweise zwei oder vier stereoskopische Beobachtungskanäle aufweisen kann. Es ist bevorzugt, das Vergrößerungssystem 7 mit vier jeweils paarweise stereoskopischen Beobachtungskanälen auszubilden, wobei jeweils ein Paar stereoskopische Beobachtungskanäle für den Hauptoperateur bzw. den Assistenten vorgesehen ist. Das Vorsehen von vier Vergrößerungskanälen im Rahmen des Vergrößerungssystems ermöglicht die Realisierung eines geringen vertikalen Abstandes zwischen jeweiliger Beobachtungsachse und dem zu beobachtenden Objekt sowohl für den Hauptoperateur als auch den Assistenten. Zweckmäßigerweise verlaufen zwei Vergrößerungskanäle des Vergrößerungssystems, insbesondere die Vergrößerungskanäle für den Hauptoperateur, horizontal auf gleicher Höhe, wobei zwei weitere Vergrößerungskanäle parallel hierzu, d. h. ebenfalls horizontal, mit einer vertikalen Beabstandung zu einander verlaufen. Diese Vergrößerungskanäle mit vertikaler Beabstandung sind insbesondere für den Assistenten nutzbar. Hierbei ist es insbesondere möglich, dass die vertikal beabstandeten Vergrößerungskanäle oberhalb bzw. unterhalb des Mittelpunkts der Verbindungslinie zwischen den auf gleicher Höhe ausgebildeten Vergrößerungskanälen für den Hauptoperateur verlaufen. Hierdurch ist eine besonders dichte Packung der vier Vergrößerungskanäle gegeben, wodurch eine besonders geringe Bauhöhe des erfindungsgemäßen Stereomikroskops realisierbar ist. In den Figuren 1 und 2 sind, aus Gründen der Anschaulichkeit, lediglich einfache Beobachtungsstrahlengänge dargestellt. Insbesondere ist der Beobachtungsstrahlengang in der zweiten Mikroskopebene II mit 12d bezeichnet. Es sei zur Erläuterung angemerkt, dass die zwei Beobachtungsstrahlengänge für den Hauptoperateur in der Beobachtungsrichtung der Figuren 1 und 2 hinter einander liegen, so dass lediglich einer dieser Beobachtungsstrahlengänge darstellbar ist. Die vertikal beabstandeten Beobachtungsstrahlengänge in der zweiten Mikroskopebene, welche an dem Umlenkelement 9 in die dritte Mikroskopebene III abgelenkt werden, sind nicht im Einzelnen dargestellt. Auch der vertikal verlaufende Beobachtungsstrahlengang 12g stellt bezüglich der bevorzugten Ausführungsform des Vergrößerungssystems 7 lediglich eine schematische Vereinfachung dar, da tatsächlich bei dieser Ausführungsform in der Darstellung der Figuren 1 und 2 insgesamt zwei nebeneinander vertikal verlaufende Beobachtungsstrahlengänge in die dritte Mikroskopebene abgelenkt werden. Eine vollständige Darstellung dieser bevorzugten Ausführungsform eines Vergrößerungssystems ist in der DE 102 55 960 offenbart, auf die hiermit Bezug genommen wird.

Mittels (nicht dargestellter) Binokulartuben ist anschließend an die Auskopplungseinrichtung 9 eine stereoskopische Beobachtung des Objekts 40 durch den Hauptoperateur bzw. den Assistenten möglich.

Zweckmäßigerweise ist zur weiteren Umlenkung der stereoskopischen Beobachtungsstrahlengänge für den Hauptoperateur hinter der Auskopplungseinrichtung 9 ein weiteres Umlenkelement 6 vorgesehen, mittels dessen die Beobachtungsstrahlengänge (bei 12e) für den Hauptoperateur von der zweiten Mikroskopebene II beispielsweise zurück in die erste Mikroskopebene I ablenkbar sind. In der ersten Mikroskopebene I ist ein weiteres Umlenkelement 16 vorgesehen, mittels dessen die Beobachtungsstrahlengänge für den Hauptoperateur wieder im Wesentlichen in eine horizontale Richtung abgelenkt werden. Die Strahlengänge zu einem (nicht dargestellten) Binokulartubus in der Mikroskopebene I sind mit 12f bezeichnet.

Ist hingegen eine Beobachtung des Objekts 40 durch den Hauptoperateur in der zweiten Mikroskopebene II gewünscht, kann auf das Umlenkelement 6 verzichtet werden, bzw. kann dieses aus dem Strahlengang verschiebbar oder halbdurchlässig ausgebildet sein. In diesem Fall ergeben sich die mit 12h bezeichneten Beobachtungsstrahlengänge für den Hauptoperateur.

Für den Assistenten ist in der dritten Mikroskopebene III ein weiteres Umlenkelement 10 vorgesehen, mittels dessen die durch die Auskopplungseinrichtung 9 ausgekoppelten Strahlengänge in die dritte Mikroskopebene (d. h. im Wesentlichen in eine horizontale Richtung) ablenkbar sind. Das Umlenkelement 10 ist zweckmäßigerweise je nach Orientierung der Assistenten-Beobachtungsstrahlengänge um eine Achse 13 oder eine zu dieser Achse senkrecht verlaufende Achse verschwenkbar, so dass ein Assistenteneinblick über den (nicht dargestellten) Assistenten-Binokulartubus im dargestellten Beispiel in die Zeichenebene hinein, oder aus der Zeichenebene heraus möglich ist.

Ein Beleuchtungssystem des dargestellten Mikroskops ist insgesamt mit 3, 4 bezeichnet, wobei mit 4 ein Faserkabel für eine Beleuchtungseinrichtung 3 bezeichnet ist. Über ein Umlenkelement 3a wird Licht aus dem Faserkabel in einem gewünschten Winkel auf das zu beleuchtende Objekt 40 aufgebracht.

Das Mikroskop 100 ist ferner mit einer Zusatzoptik 30, 32 ausgestattet, welche die Durchführung intraokularer Chirurgie ermöglicht.

Die Zusatzoptik weist eine Ophthalmoskopierlinse bzw. Funduslinse 30 und eine Korrekturlinse 32 auf. Die Ophthalmoskopierlinse 30 dient zum Ausgleich der Brechkraft des Auges.

Da die Ophthalmoskopierlinse 30 und die Korrekturlinse 32 bei intraokularer Chirurgie gemeinsam verwendet werden, sind sie zweckmäßigerweise mittels eines nicht dargestellten Verschwenkmechanismus aus dem Strahlengang 12a zwischen Objekt 40 und Hauptobjektiv 2 bzw. der optischen Achse 11 des Hauptobjektivs 2 heraus verschwenkbar. Durch diese Verschwenkbarkeit ist gewährleistet, daß das Mikroskop 100 auch für andere chirurgische Eingriffe, welche keine derartige Zusatzoptik benötigen, eingesetzt werden kann.

Zur Funktionsweise der Zusatzoptik sei angemerkt, daß die Ophthalmoskopierlinse 30 eine erste Zwischenabbildung 31 des Objekts 40 vor dem Hauptobjektiv 2 des Mikroskops 100 erzeugt. Das durch die Ophthalmoskopierlinse 30 erzeugte Bild 31 ist höhen- und seitenverkehrt. Die Korrekturlinse 32 ist zweckmäßigerweise entlang der optischen Achse 11 verschiebbar ausgebildet, wie mittels Doppelpfeil angedeutet ist. Durch Verschiebung der Korrekturlinse 32 ist es beispielsweise möglich, auf einen interessierenden Abschnitt des Objektes bzw. Auges 40 zu fokussieren, ohne an den optischen Systemen im Gehäuse 102 Einstellungen vornehmen zu müssen.

Da das Zwischenbild 31 seitenverkehrt und höhenverkehrt und damit in der Beobachtung pseudostereoskopisch ist weisen die Zusatzkomponenten 8 SDI-Elemente auf, welche diesen Effekt korrigieren. Derartige SDI-Elemente bestehen aus einem relativ komplexen Satz von Prismen. Derartige Systeme sind an sich bekannt und bedürfen, da nicht erfindungswesentlich, an dieser Stelle keiner weiteren Erläuterung.

Es ist ebenfalls möglich, ein derartiges höhen- und seitenverkehrtes und pseudostereoskopisches Bild mit Hilfe einer Linsenabbildung wieder in ein seitenrichtiges und höhenrichtiges Bild zu transformieren. Eine derartige Linsenoptik, die aus mindestens zwei Linsen bestehen muß, baut jedoch relativ lang.

Eine bevorzugte Ausführungsform des erfindungsgemäßen Mikroskops wird nun anhand der Figur 2 erläutert.

Das insgesamt mit 200 bezeichnete Mikroskop gemäß Figur 2 unterscheidet sich von dem Mikroskop 100 gemäß Figur 1 lediglich in der Zusatzoptik sowie der Ausbildung der Umlenkelemente zur Umlenkung des Strahlenganges aus der ersten Mikroskopebene I in die zweite Mikroskopebene II. Die übrigen Komponenten des Mikroskops entsprechen denjenigen gemäß Mikroskop 100 der Figur 1, und sind daher auch mit gleichen Bezugszeichen bezeichnet. Auf eine nochmalige ausführliche Beschreibung dieser Komponenten bzw. der im Mikroskop realisierten Strahlengänge wird daher verzichtet.

Zunächst erkennt man, daß, wie auch bei dem Mikroskop gemäß Figur 1, die Ophthalmoskopierlinse 30a dem Hauptobjektiv 2 vorgeschaltet ist. Eine Korrekturlinse, hier mit 32a bezeichnet, ist jedoch dem Hauptobjektiv 2 nachgeschaltet, d.h. im Mikroskopgehäuse 202 angeordnet. Die Korrekturlinse 32a ist aus dem das Hauptobjektiv 2 durchsetzenden Beobachtungsstrahlengang 12a entfernbar, wie mittels des Doppelpfeils P angedeutet ist. Ferner ist die Korrekturlinse 32a entlang der optischen Achse 11 des Hauptobjektivs 2 bzw. dem Beobachtungsstrahlengang 12a verschiebbar, wie mittels des Doppelpfeiles Q angedeutet ist. Die Entfernbarkeit der Korrekturlinse 32a ermöglicht, bei gleichzeitiger Verschwenkung oder Entfernung der Funduslinse bzw. Ophthalmoskopierlinse 30a aus dem Beobachtungsstrahlengang durch das Hauptobjektiv 2, die Verwendung des Mikroskops 200 für beliebige ophthalmologische Anwendungen. Durch die Verschiebbarkeit der Korrekturlinse 32a parallel zur optischen Achse 11 ist eine Fokussierung des das Hauptobjektiv 2 beaufschlagenden Strahlenganges 12a möglich.

Dadurch, daß die Korrekturlinse 32a nun erfindungsgemäß innerhalb des Mikroskopgehäuses 202 angeordnet ist, kann eine Verschmutzung während einer Operation ausgeschlossen werden. Die einzige Linse, welche erfindungsgemäß noch der Gefahr einer Verschmutzung während einer Operation ausgesetzt ist, ist die Ophthalmoskopierlinse 30a, welche bereits aufgrund ihrer kleinen Größe relativ einfach und unaufwändig zu säubern oder auszutauschen ist.

Wie bereits angedeutet, dient die Korrekturlinse 32a zur Fokussierung des Beobachtungsstrahlenganges durch das Hauptobjektiv 2 auf das Zwischenbild 31. Es ist, gemäß einer weiteren, nicht dargestellten Ausführungsform des Mikroskops 200 möglich, auf die Korrekturlinse 32a zu verzichten, und sowohl die entsprechende Brechkraft als auch Fokussierbarkeit innerhalb bzw. mittels der optischen Zusatzkomponenten 8 zu verwirklichen.

Zweckmäßigerweise wird hierbei ein optisches Element der Zusatzkomponenten 8, bspw. eine Linse, bezüglich der hier mit 11a, 11b bezeichneten optischen Achsen der ersten bzw. zweiten Mikroskopebene I bzw. II verschwenkbar bzw. verschiebbar ausgebildet. Über eine entsprechende Verschiebbarkeit ist auch hier eine Fokussierung möglich. Mit dieser Lösung, bei der die Funktion der Korrekturlinse 32a mittels einer optischen Komponente im Rahmen der Zusatzkomponenten 8 verwirklicht wird, ist die Bauhöhe des Mikroskops reduzierbar, da die optische Achse der Zusatzkomponenten 8 in der horizontalen Ebene I bzw. II verläuft.

Es ist ebenfalls möglich, eine Aufteilung der Funktionen der Korrekturlinse 32a, d. h. der Bereitstellung der Brechkraft und der Fokussierung, auf mehrere Linsen zu realisieren. Es ist beispielsweise denkbar, die Linse 32a in der in Figur 2 dargestellten Position in Richtung der optischen Achse 11 nicht verschiebbar auszubilden. Eine derartige Linse erfüllt nur die Funktion der Bereitstellung der Brechkraft. Die Fokussierung kann dann im Rahmen der Zusatzkomponenten 8 durch entsprechende Verschiebbarkeit einer Linse entlang der optischen Achse 11a in der ersten Ebene I oder der optischen Achse 11b in der zweiten Ebene II realisiert werden. Es ist ebenfalls denkbar, beispielsweise das Umlenkelement 5 mit einer Brechkraft zu versehen. Hier ist es beispielsweise möglich, das Umlenkelement in Form eines Hohlspiegels oder mittels eines gekrümmte Flächen aufweisenden Prismas zu realisieren (in der Figur 2 nicht explizit dargestellt).

Es sei ferner angemerkt, dass es ebenfalls möglich ist, zwischen dem Hauptobjektiv 2 und der dem Hauptobjektiv 2 nachgeschalteten Komponente 32a das Umlenkelement 5 vorzusehen. Dies bedeutet eine Anordnung der optischen Komponente 32a in der ersten Mikroskopebene I bzw. entlang der optischen Achse 11a. Diese Ausführungsform ist in Figur 2 jedoch nicht dargestellt.

Auch die in Figur 2 mit 22a, 22b bezeichneten Umlenkelemente 21a, 21b sind bevorzugt als Hohlspiegel oder gekrümmte Flächen aufweisende Prismen realisierbar. Mit dieser Maßnahme ist es beispielsweise möglich, eine Zwischenabbildung 22 entlang des senkrecht verlaufenden Strahlenganges 12c zwischen den Mikroskopebenen I, II zu erzeugen. Somit kann auch dieser vertikal verlaufende Abschnitt zur optischen Bearbeitung des Strahlenganges ausgenutzt werden, wodurch die horizontale Erstreckung des Mikroskops 200 verkleinert werden kann. Insbesondere ist wie erwähnt, das Zwischenbild 31 hinter der Ophtalmoskopierlinse 30a seitenverkehrt und höhenverkehrt und damit in der Beobachtung pseudostereoskopisch. Die als Hohlspiegel ausgebildeten Umlenkelemente 22a, 22b sind in der Lage, aus diesem pseudostereoskopischen Bild ein höhen- und seitenrichtiges Bild zu erzeugen, d.h. als Inverter zu fungieren. Im Einzelnen ergibt sich folgende Beobachtungsstrahlpropagierung: Die aus dem höhenund seitenverkehrten Zwischenbild 31 resultierenden Strahlengänge werden mittels der Korrektur- bzw. Hilfslinse 32a oder gegebenenfalls (nach Umlenkung am Umlenkelement 5) der optischen Zusatzkomponenten 8 in einen im Wesentlichen achsparallelen Strahlengang entlang der optischen Achse 11a der ersten Mikroskopebene I umgewandelt. Dieser achsparallele Strahlengang wird mittels des Hohlspiegels 21a in die weitere Zwischenabbildung 22 im vertikalen Strahlengang 12c zwischen den beiden Mikroskopebenen I, II abgelenkt. Diese Zwischenabbildung 22 ist seitenrichtig und höhenrichtig und stereoskopisch. Diese Zwischenabbildung 22 wird dann mittels des Hohlspiegels 22b in der zweiten Mikroskopebene II wieder ins Unendliche abgebildet (im Wesentlichen achsparalleler Strahlengang). Entlang der optischen Achse 11b der zweiten Mikroskopebene II befindet sich das vorzugsweise als vierkanaliges Zoom-System ausgebildete Vergrößerungssystem 7, wodurch, wie bereits erwähnt die stereoskopische Aufspaltung für den Hauptoperateur und Assistenten stattfindet. Es sei an dieser Stelle auf die Doppelfunktionalität der Umlenkelemente 22a, 22b verwiesen. Einerseits dienen sie zur Umlenkung der Strahlengänge und somit zur optimalen Raumausnutzung innerhalb des Mikroskopkörpers 202, andererseits zur Invertierung eines pseudostereoskopischen Zwischenbildes, wodurch die Anzahl der optischen Komponenten gegenüber herkömmlichen Lösungen reduzierbar ist.

Die Umlenkelemente 22a, 22b dienen also jeweils sowohl zur Umlenkung der Beobachtungsstrahlengänge innerhalb des Mikroskopgehäuses als auch zur Bilderzeugung bzw. zur Abbildung ins unendliche, wodurch in einfacher und preiswerter Weise eine Bildaufrichtung einer invertierten, pseudostereoskopischen Zwischenabbildung zur Verfügung gestellt ist.

Gemäß der dargestellten Ausführungsform ist es also möglich, herkömmlich verwendete SDI-Systeme, welche relativ komplexe Prismensysteme aufweisen, durch einfache Hohlspiegel 21a, 21b zu ersetzen. Es ist ebenfalls denkbar, die durch die Hohlspiegel 22a, 22b bereitgestellte Doppelfunktionalität mittels mit einer Brechkraft ausgebildeter Umlenkprismen bereit zu stellen. Es wäre ebenfalls denkbar, anstelle des Umlenkelements 22a oder 22b das Umlenkelement 5 mit einer Brechkraft auszubilden. Hierdurch würde das Zwischenbild 22 in der ersten Mikroskopebene I erzeugt werden.

Durch die Positionierung eines mit vier Beobachtungskanälen ausgebildeten Vergrößerungssystems hinter den als Hohlspiegel ausgebildeten Umlenkelementen 22a, 22b ist es möglich, sowohl für einen Hauptoperateur als auch einen Assistenten eine stereoskopische Beobachtung bereit zu stellen, wobei lediglich ein Inverter-System (vorliegend gegeben durch die Umlenkelemente 22a, 22b) notwendig ist. Die Bereitstellung lediglich eines Inverter-Systems für Hauptoperateur und Assistenten ist auch dann realisierbar, wenn das Vergrößerungssystem vor dem Inverter-System (d.h. objektivseitig) angeordnet wird. Es muss jedoch vor einer Auskopplungseinrichtung (in Fig. 2 mit 9 bezeichnet) angeordnet sein, mittels welcher die Strahlengänge für Hauptoperateur und Assistent in unterschiedliche Richtungen gelenkt werden.

Insgesamt sind somit sämtliche optische Komponenten innerhalb des Mikroskopkörpers 202 vorgesehen, mit Ausnahme der Ophthalmoskopierlinse 30a. Diese Linse 30a ist, wie erläutert, mechanisch mit dem Mikroskop verbunden und verschwenkbar gehaltert, sowie elektromechanisch mit der Korrekturlinse 32a verbunden.

Der Vollständigkeit halber sei ferner darauf hingewiesen, dass eine Dateneinspiegelung beispielsweise an der Stelle des Umlenkelements 6 oder an anderer geeigneter Stelle vorgesehen sein kann. Hier kann beispielsweise auch ein optischer Strahlenteiler, bspw. für eine Dokumentationseinrichtung, eingebracht werden.

Es sei bzgl. der Beleuchtungseinrichtung 3 schließlich darauf hingewiesen, dass gemäß den vorliegend dargestellten Ausführungsformen die Beleuchtung zwischen der Korrekturlinse 32 bzw. 32a und dem Vergrößerungssystem 7 eingespiegelt wird. Dies führt zu dem vorteilhaften Effekt, dass das Leuchtfeld stets die richtige Größe (korrekter Durchmessers) bzgl. einer beliebig eingestellten Fokussierung mittels der Korrekturlinse 32 bzw. 32a aufweist.

### Bezugszeichenliste:

- 2: Hauptobjektiv
- 3: Beleuchtungseinrichtung
- 3a: Umlenkelement
- 4: Faserkabel
- 5,6: Umlenkelemente
- 7: Vergrößerungssystem (Zoomsystem)
- 8: optische Komponenten
- 9: Umlenkelement bzw. Auskopplungseinrichtung
- 10: Umlenkelement
- 11: optische Achse des Hauptobjektivs 2
- 11a, 11b: optische Achse der ersten bzw. zweiten Mikroskop ebene
- 12a - 12h: Beobachtungsstrahlengänge
- 13: Schwenkachse des Umlenkelements 10
- 16: Umlenkelement
- 21a, 21b: Umlenkelemente
- 22a, 22b: Umlenkelemente (Hohlspiegel, Inverter-System)
- 22: Zwischenabbildung
- 30: Ophthalmoskopierlinse bzw. Funduslinse
- 30a: Ophthalmoskopierlinse bzw. Funduslinse
- 31: Zwischenabbildung
- 32: Korrekturlinse
- 32a: Korrekturlinse
- 40: Objekt
- 100: Stereomikroskop
- 102: Gehäuse (Mikroskopkörper)
- 200: Stereomikroskop
- 202: Gehäuse
- I, II, III: Mikroskopebenen

## Patentansprüche

1. Mikroskop, insbesondere Stereomikroskop, mit einem Hauptobjektiv (2), einem diesem nachgeschalteten Vergrößerungssystem (7) und einer Zusatzoptik (30, 32a) zur Durchführung intraokularer Chirurgie,
wobei die Zusatzoptik wenigstens eine dem Hauptobjektiv (2) vorgeschaltete Ophthalmoskopierlinse (30a) und wenigstens eine dem Hauptobjektiv (2) nachgeschaltete optische Komponente (32a) zur Bereitstellung einer Brechung und Fokussierung eines das Hauptobjektiv (2) durchsetzenden Beobachtungsstrahlenganges aufweist, **dadurch gekennzeichnet, dass** die optische Achse (12d) des dem Hauptobjektiv (2) nachgeschalteten Vergrößerungssystems (7) im wesentlichen senkrecht zu der optischen Achse (11) des Hauptobjektivs (2) verläuft.

2. Mikroskop nach Anspruch 1, **dadurch gekennzeichnet, dass** die dem Hauptobjektiv (2) vorgeschaltete Ophthalmoskopierlinse (30a) und die dem Hauptobjektiv (2) nachgeschaltete optische Komponente (32a) entlang der optischen Achse (11) des Hauptobjektivs angeordnet sind.

3. Mikroskop nach einem der Ansprüche 1 oder 2, **dadurch gekennzeichnet, dass** das Vergrößerungssystem (7) wenigstens zwei stereoskopische Beobachtungskanäle aufweist.

4. Mikroskop nach einem der vorstehenden Ansprüche, **dadurch gekennzeichnet, daß** die dem Hauptobjektiv (2) vorgeschaltete Ophthalmoskopierlinse (30a) der Zusatzoptik, insbesondere durch Verschwenkung, und/oder die dem Hauptobjektiv (2) nachgeschaltete optische Komponente (32a), insbesondere durch Verschiebung, aus dem Beobachtungsstrahlengang des Hauptobjektivs (2) entfernbar ist.

5. Mikroskop nach einem der vorstehenden Ansprüche, **dadurch gekennzeichnet, daß** die dem Hauptobjektiv (2) nachgeschaltete Komponente (32a) der Zusatzoptik in Richtung des sie beaufschlagenden Beobachtungsstrahlengangs hin- und herverschiebbar ist.

6. Mikroskop nach einem der vorstehenden Ansprüche, **gekennzeichnet durch** ein Umlenkelement (5), das einen entlang der optischen Achse (11) des Hauptobjektivs verlaufenden Strahlengang in eine erste, sich im Wesentlichen senkrecht zu der optischen Achse (11) des Hauptobjektivs erstreckenden Mikroskopebene (I) umlenkt.

7. Mikroskop nach Anspruch 6, **gekennzeichnet durch** zwei Umlenkelemente (22a, 22b), mittels der ein in der ersten Mikroskopebene (I) verlaufender Strahlengang in eine zweite Mikroskopebene (II) ablenkbar ist, wobei wenigstens eines der Umlenkelemente (22a, 22b) mit einer Brechkraft ausgebildet ist.

8. Mikroskop nach Anspruch 6, **dadurch gekennzeichnet, dass** das Umlenkelement (5) zwischen dem Hauptobjektiv (2) und der dem Hauptobjektiv (2) nachgeschalteten optischen Komponente (32a) der Zusatzoptik vorgesehen ist.

9. Mikroskop nach einem der Ansprüche 6 bis 8, **dadurch gekennzeichnet, dass** das Umlenkelement (5) mit einer Brechkraft ausgebildet ist.

10. Mikroskop nach einem der vorstehenden Ansprüche, **dadurch gekennzeichnet, dass** die Ophthalmoskopierlinse (30a) und die optische Komponente (32a) zur Ermöglichung eines gemeinsamen Ausschwenkens bzw. Verschiebens bezüglich des Strahlenganges elektromechanisch miteinander verbunden sind.

11. Mikroskop nach einem der vorstehenden Ansprüche, **dadurch gekennzeichnet, dass** die Brechkraft und der Verschiebebereich der optischen Komponente (32a) so gewählt sind, dass ein Fokussierausgleich vom Ort der Objektbeobachtung bei Nicht-Verwendung der Ophthalmoskopierlinse (30a) bis zum Ort der Zwischenabbildung (31) möglich ist.

12. Mikroskop nach einem der vorstehenden Ansprüche, **gekennzeichnet durch** ein Autofokussystem zur Steuerung der Verschiebung der optischen Komponente (32a) parallel zur optischen Achse (11) des Hauptobjektivs (2).
